# EUROPEAN PATENT APPLICATION

(11) **EP 1 176 211 A1**
(43) Date of publication of application: **30.01.2002**
(21) Application number: 00116070.4
(22) Date of filing: 27.07.2000
(51) Int. Cl.: C12Q 1/68, C12N 15/82

(54) **Process for monitoring mutagens in plants**

(71) Applicant: Novartis Forschungsstiftung, c/o Novartis International AG, 4056 Basel (CH)
(72) Inventor: Hohn, Barbara, 4144 Arlesheim (CH); Kovalchuk, Olga, 4132 Muttenz (CH); Kovalchuk, Igor, 4132 Muttenz (CH)
(74) Representative: Blum, Rudolf Emil Ernst

(57) **Abstract**

A process for monitoring mutagens or a test compound, in particular at low concentrations, is disclosed. Organisms comprising a nucleic acid with at least one point mutation resulting in loss or reduction in funtion of a reporter molecule are contacted with a mutagen or a test compound and the presence of a functional reporter molecule as an indicator of reversion of said point mutation is detected.

## Description

The present invention relates to a process for monitoring mutagens as well as modified cells, and in particular plants, and their use as bioindicators of environmental pollution.

Life on earth is facing more and more environmental pollution. With the appearance of new toxicants new methods are required for the evaluation of their possible hazardous influence on living organisms.

The available methods using plants as biosensors of genetic toxicity of environmental pollutants are based on the detection of chromosomal aberrations in the plant genome. The *Allium cepa* chromosomes aberration test provides a rapid screen for toxic effects of chemicals. The assay is based on the evaluation of the percentage of cells undergoing mitosis, the estimation of the number of aberrant versus normal mitotic events and different fractions of chromosomal aberrations (Kovalchuk et al., 1999).

Another plant useful for environmental mutagenesis studies is Tradescantia. This plant allows three different bioassays to be perfomed: 1. a chromosome aberration assay based on the observation of visible chromosome damage during mitosis in roottips, pollen-tubes and microspores; 2. a stamen-hair mutation assay; and 3. a cytogenetic test based on the formation of micronuclei that results from chromosome breakage in the pollen mother cells (Kovalchuk et al., 1999).

Although the process described above can be applied to detect certain concentrations of mutagens, they fail to detect mutagenic influence at low concentrations of mutagens.

In Kovalchuk et al., 1998 transgenic A. thaliana plants and their use as bioindicators of radioactive waste are described. The plants carry two overlapping, nonfunctional truncated versions of a chimeric β-glucuronidase (uidA) marker gene in inverted orientation as a recombination substrate. In cells in which events of homologous recombination at this transgenic locus have occurred the uidA gene was restored. This transgenic system enables the quantitative visualization of intrachromosomal events in the marker transgene induced by radiation damage.

There exists a great need in providing processes and means allowing the detection of low concentrations of mutagens e.g. heavy metals and organic substances and radiation.

Hence it is an object of the present invention to provide a method for monitoring mutagens, in particular at very low concentrations, said method comprising contacting an organism, a cell or a protoplast with a mutagen or a test compound, said organism or cell comprising a nucleic acid with at least one point mutation resulting in a detectable change in function and/or expression of a reporter molecule
and detecting the presence of said functional reporter molecule as an indicator of reversion of said point mutation.

The plant, plant cell or protoplast used in the method of the invention preferably comprises:
(I) a nucleic acid encoding a reporter molecule, said nucleic acid having at least one point mutation resulting in a non-functional reporter molecule and/or
(II) a nucleic acid encoding a repressor and a nucleic acid encoding a reporter molecule, wherein said nucleic acid encoding said repressor comprises at least one point mutation affecting repressor activity and wherein said repressor produced in the absence of said point mutation inhibits expression of said reporter molecule, and/or
(III) a nucleic acid with at least one intron in a reporter-coding sequence, said intron or intron-exon boundary comprising at least one point mutation introducing an alternative splice site into said nucleic acid thereby affecting expression of a functional reporter molecule, and/or
(IV) a nucleic acid encoding a reporter molecule, operably linked to a promoter, wherein said promoter comprises at least one point mutation affecting expression of said reporter molecule.

It is a further object of the present invention to provide an organism, a plant cell or a protoplast comprising a nucleic acid with at least one point mutation resulting in a detectable change in function and/or expression of a reporter molecule.

The organism, plant cell or protoplast of the present invention preferably comprises:
(I) a nucleic acid encoding a reporter molecule, said nucleic acid having at least one point mutation resulting in a non-functional reporter molecule and/or
(II) a nucleic acid encoding a repressor and a nucleic acid encoding a reporter molecule, wherein said nucleic acid encoding said repressor comprises at least one point mutation affecting repressor activity and wherein said repressor produced in the absence of said point mutation inhibits expression of said reporter molecule, and/or
(III) a nucleic acid with at least one intron in a reporter-coding sequence, said intron or intron-exon boundary comprising at least one point mutation introducing an alternative splice site into said nucleic acid thereby affecting expression of said reporter molecule, and/or
(IV) a nucleic acid encoding a reporter molecule, operably linked to a promoter, wherein said promoter comprises at least one point mutation affecting expression of said reporter molecule;

In a first specific embodiment, the invention provides a method for monitoring mutagens, in particular at very low concentrations, said method comprising contacting an organism or a cell with a mutagen or a test compound, said organism or cell having a nucleic acid encoding a reporter molecule, said nucleic acid having at least one point mutation resulting in a non-functional reporter molecule.

Preferred organisms are plants including their reproduction material and preferred cells are plant cells or protoplasts. Plants have the specific advantage that they can be planted on a soil of interest indicating the "quality" of the whole planted area and not only of specific samples. Reproduction materials include stems, tubers, leafs, cells and also calli.

For the purposes of the present invention a reporter molecule-encoding nucleic acid can be any nucleic acid encoding a polypeptide product which is detectable by means of a positive indicator e.g. a colour change, survival, fluorescence or resistance. Suitable marker molecules are for example β-glucuronidase, green fluorescent protein (GFP) or other fluorescent proteins, luciferase, R locus in maize, galactosidase, herbicide resistance, fungicide resistance, kanamycin resistance, hygromycin resistance etc. Fluorescent proteins, such as GFP are especially useful for automatisation in toxicity testing as robotics can be used to identify GFP expressing cells, for example, and even to count the number of fluorescent cells, i.e. to quantify.

For the purposes of the present invention any reporter molecule-encoding nucleic acid that can be expressed in a eucaryotic cell, preferably in a plant cell or protoplast, can serve as starting material for the introduction of at least one point mutation leading to a nucleic acid encoding a non functional reporter molecule. Nucleic acids suitable as starting material are all nucleic acids encoding a reporter molecule or a fragment thereof having similar biological activity. Such nucleic acids can be naturally occurring genomic DNA or cDNA, for example, or nucleic acids that are identical with such naturally occuring nucleic acids but for the degeneracy of the genetic code as well as nucleic acids encoding fragments and mutations provided that the encoded polypeptides have reporter molecule activity. Said encoding nucleic acids, after point mutation, in particular after site directed mutagenesis, can either be introduced into a suitable environment of the genome of a cell or a protoplast to be transformed enabling the expression of reporter molecule or they can be introduced into the genome of the cell or the protoplast together with suitable regulatory sequences, such as their natural or adapted regulatory sequences. Said reporter molecule-encoding nucleic acid can also be expressed from an extrachromosomal gene construct e.g. from an episomal vector enabeling expression of the reporter gene in a host cell or a protoplast. The nucleic acid can be introduced into the cell or a protoplast by any transformation method leading to uptake of the nucleic acid sequence into the cell.

Any point mutation in a nucleic acid encoding a reporter molecule resulting in an inactive form and correctable to a functional activity can be used in the methods of the present invention. Point mutations leading to amino acid substitutions which result in an inactive reporter activity are preferred, in particular point mutations leading to nonsense codons causing premature termination of protein synthesis. The sequence context of the generated stop codon is optimally taken into consideration to prevent leakage of the generated stop codon. Preferably a stop codon is followed by adenine or guanine, which have been shown to promote efficient termination of translation. The leakage of the stop codon mutations is optimally low in comparison to the expression of reverted nucleic acids to prevent background reporter gene activity. The resulting polypeptides have reduced reporter gene activity, but preferably no or background reporter gene activity, and the nucleotide changes preferably comprise C-T, G-T, A-T and G-A nucleotide changes.

Mutations in naturally occuring genes that result in a change of phenotype can also be used. For example, dwarfism in wheat where correction of mutation results in tall plants compared to short plants; albinism (chlorophyll deficiency) where correction results in green plants instead of white plants.

The reversion frequency of the introduced mutations are preferably high enough to yield scorable events in reasonably sized plant populations.

In contrast to other known systems using plants as bioindicators, the design of the process and the organism or cells or protoplasts of the present invention allow the identification of specific classes of mutations, nameley T-C, T-A, T-G or A-G mutations. The A-G mutation is of particular interest since it is one of the major types of mutation which cause cancer development in mammals. Therefore the present invention provides means for detecting mutagens causing mutations, in particular mutations that are known to lead to cancer development.

In another embodiment of the present invention a repressor system is used for monitoring mutagens. In said system a nucleic acid encoding a reporter molecule is under the transcriptonal control of a promoter having a binding site for a repressor. At least one point mutation is introduced in the coding or regulatory sequence of the repressor resulting in a non-functional repressor or repressor activity. Upon reversion to provide a functional repressor or repressor activity, expression of said reporter gene can be detected. Any repressor system that is functional in an eucaryotic cell, preferably in a plant cell or a protoplast, is suitable as a repressor system for example the Tet operator (TetR), which binds to tet repressor sequences in the absence of tetracycline and represses gene transcription and the lacI/lacZ system.

In another embodiment of the present invention an alternative splicing system is used for monitoring mutagens. A nucleic acid encoding a reporter with at least one intron within the reporter-coding sequence is used for monitoring mutagens. One or more point mutations can be introduced at any of the four nucleotides "AG" of the 5' exon boundary or "GT" of 3' exon boundary, thereby abolishing splicing and resulting in a non-functional reporter protein. Preferably, at least one point mutation, more preferably 3-5, most preferably 5-10 point mutations are introduced into the intron, provided that they do not interfere with the spliceosome binding site, where said at least one point mutation results in aberrant splicing, and whereby, preferably correction of one point mutation would be sufficient to restore correct splicing and production of functional reporter protein. The method is not limited to the use of introns naturally occuring in tandem with the coding sequence, as these can be replaced by synthetic or otherwise non-naturally occuring introns (i.e. not naturally occuring in tandem with the coding sequence) to form chimaeric sequences. In addition, a synthetic or non-naturally occuring intron can be introduced into cDNA or other coding sequence without introns.

In a further embodiment of the present invention a promoter based system is used for monitoring mutagens. In this case a reporter gene can be under control of a promoter harboring at least one point mutation, preferably in an essential promoter element, resulting in negligible expression of the marker gene. Upon reversion to provide functional promoter activity, an elevated expression of marker protein can be detected. Any promoter element that can be inactivated by introduction of at least one point mutation can be used for a process in accordance with this embodiment of the present invention. Suitable promoters are for example viral promoters e.g. CaMV 35S, 19S), Ti plasmid promoters e.g. nopaline synthase, plant promoters e.g. actin, ubiquitin.

In a process of the present invention any eucaryotic cell can be used, preferably plant cells or protoplasts. Plant cells, protoplasts or plants of any species can be used e.g. A. thaliana, tobacco, Brassica sp., wheat, maize, rice, Nicotiana, barley. Although whole plants are preferred, especially for environmental testing, protoplasts, plant cells and plant tissues can be used. In particular, for toxicity testing of drugs, a more sterile environment might be envisioned using protoplasts, especially in conjunction with fluorescence measurement and automatisation.

The detection of restored reporter molecule activity due to reversion of a mutated nucleotide can be done by methods well-known to a person skilled in the art. Said methods comprise for example, spectrophotometric, fluorometric and histochemical methods, and selection for resistance or other phenotype.

The process of the present invention involving point-mutation based bioindicators is suitable for detecting low levels of mutagens in e.g. polluted soils and water and is therefore e.g. useful to control phytoremediation efforts. The term mutagen as used herein includes all kind of substances and radiation directly or indirectly inducing changes in the DNA of organisms e.g. heavy metals, organic toxicants, radioactive substances, X-rays and UV-light. The process of the present invention is also useful e.g. in toxicity testing of drugs and household chemicals.

Said process as relying on point mutation based bioindicators can be used alone or in combination with other bioindicators such as e.g. recombination based bioindicators as described below, for determining any of the above mutagens including radioactive substances.

In a further aspect of the present invention, a process for monitoring mutagens, such as heavy metals, organic or inorganic toxic compounds and/or chemical waste is provided using recombination-based transgenic plant bioindicators. In this aspect of the present invention, plants harboring a marker gene are used to monitor recombination events resulting from exposure of the plant to a mutagen. An increase in intrachromosomal recombination frequency can be observed in the presence of the mutagen by detecting the marker gene, which is not functional prior to recombination. The term recombination refers to events where DNA sequences are exchanged between the recombining molecules as well as gene conversion events where DNA sequences are exchanged in a nonreciprocal manner.

Any nucleic acid or combination of nucleic acids can be used that upon correction would lead to a measurable output. For example, a coding sequence might be interrupted by an excisable stuffer sequence that upon recombination, results in a coding sequence that can be expressed to give a functional protein. An excisable stuffer sequence is any sequence that interrupts reporter regulatory or reporter coding sequences resulting in a loss of function of the reporter. The excisable stuffer sequence can therefore be a non-encoding (e.g. nonsense) sequence or a coding sequence, such as a duplication of reporter sequences. When the reporter regulatory or coding sequences are not on the same nucleic acid strand, the excisable stuffer sequence merely flanks the reporter sequences. Similarly, the "excisable coding sequence" might be between a promoter operably linked to its coding sequence, or interrupt a promoter. Orientation of the coding sequence to the promoter sequence should be considered as is clear to one of ordinary skill in the art to ensure that upon recombination all sequences are operably linked to result in a functional reporter. In a specific embodiment of this aspect of the invention said nucleic acid or combination of nucleic acids that upon correction lead to a measurable output are part of a extrachromosomal construct e.g. an episomal vector. Suitable marker genes and promoter sequences are described above.

Thus, the present invention provides methods for sensing mutagens either alone or in combination with the point mutation plants described above. An example of a transgenic plant, which can be used to detect recombination events is provided in Kovalchuck et al., 1998. The use of such plants for detecting radiation-induced damage is not envisioned within the scope of the present invention.

Plant cells, protoplasts or plants of any species can be used e.g. Arabidopsis sp., tobacco, Brassica sp., wheat, maize, rice, Nicotiana, barley. Although whole plants are preferred, especially for environmental testing, protoplasts, plant cells and plant tissues can be used.

The invention will be better understood and objects other than those set forth above will become apparent when consideration is given to the following detailed description thereof. Such description makes reference to the annexed drawings, wherein:
Figure 1A shows stop codons generated in the open reading frame (ORF) of the E. coli β-glucuronidase gene (uidA),
Figure 1B shows stop codon reversion visualised in a 4 week old Arabidopsis thaliana plant,
Figure 2A shows frequencies of spontaneous and induced reversions in sub-lines of line 49 C-T,
Figure 2B shows frequencies of spontaneous and induced reversions in sub-lines of line 112 G-T,
Figure 2C shows frequencies of spontaneous and induced reversions in sub-lines of line 118 A-T,
Figure 2D shows frequencies of spontaneous and induced reversions in sub-lines of line 166 G-T,
Figure 2E shows frequencies of spontaneous and induced reversions in sub-lines of lines 424 G-T and 166 G-A; the numbers on the top of the bars represent copy numbers of the transgenes; * the transgenes in such star marked sub-lines are integrated in complex patterns of single or multiple loci,
Figure 2F shows average frequencies of reversions as calculated per plant per single transgene copy. Bars represent average frequencies of reversions with standard error and numbers on the top of the bars represent the fold induction,
Figure 3 shows enzymatic activity of mutant E. coli β-glucuronidase (uidA) proteins,
Figure 4 shows frequency of spontaneous and induced reversions in different Arabidopsis thaliana lines carrying mutated uidA genes,
Figure 5 shows a β-glucuronidase recombination substrate of A. thaliana plants used in a recombination assay for detecting mutagens
Figure 6 shows results of a Arabidopsis thaliana mutation assay and
Figure 7 shows results of a Arabidopsis thaliana recombination assay
Below several aspects of the present invention are disclosed in detail.

### 1. Generation of transgenic plants harboring a reporter molecule encoding nucleic acid, such as E. coli β-glucuronidase (uidA), inactivated by point mutations

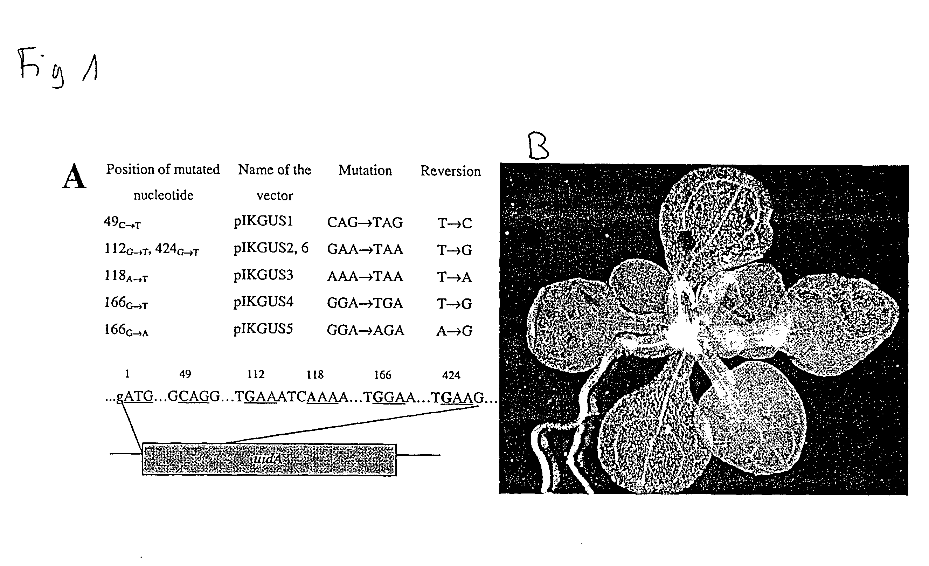

Using site-directed mutagenesis, different point mutations leading to a non functional reporter molecule, preferably point mutations leading to stop codons, can be introduced into a reporter molecule coding sequence such as β-glucuronidase; for example one or more stop codons can be introduced into any site of the uidA gene, preferably in 5 sites in the 5' proximal part of uidA. Constructs resulting from such site directed mutagenesis can be inserted into e.g. a binary vector plasmid and introduced into a plant, preferably an Arabidopsis thaliana ecotype Columbia plant (Figure 1A). In cases where a stop codon has been generated, sequence context is preferably taken into consideration to prevent leakage of the stop codon; a stop codon is preferably generated in a triplet followed by adenine or guanine. This environment had been shown to be optimal for efficient termination of translation (Atkinson and Martin, 1994; Betzner et al., 1997). Reversion to the original nucleotide, or mutation to another amino acid that allows protein function was found to restore the activity of the gene. In addition a construct causing an amino-acid substitution, such as substitution GGA to AGA in position 166, coding for Gly in place of Arg, can be chosen for plant transformation (Figure 1A). This allows to study the frequency of reversions of A to G in the case of amino acid substitution, in addition to the mutations from T to C, A or G in the case of stop codon mutation.

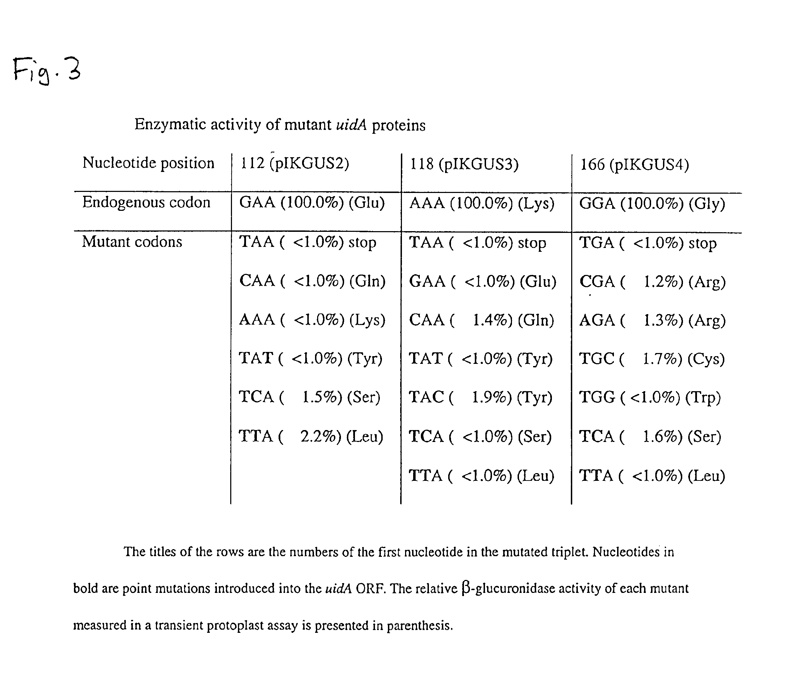

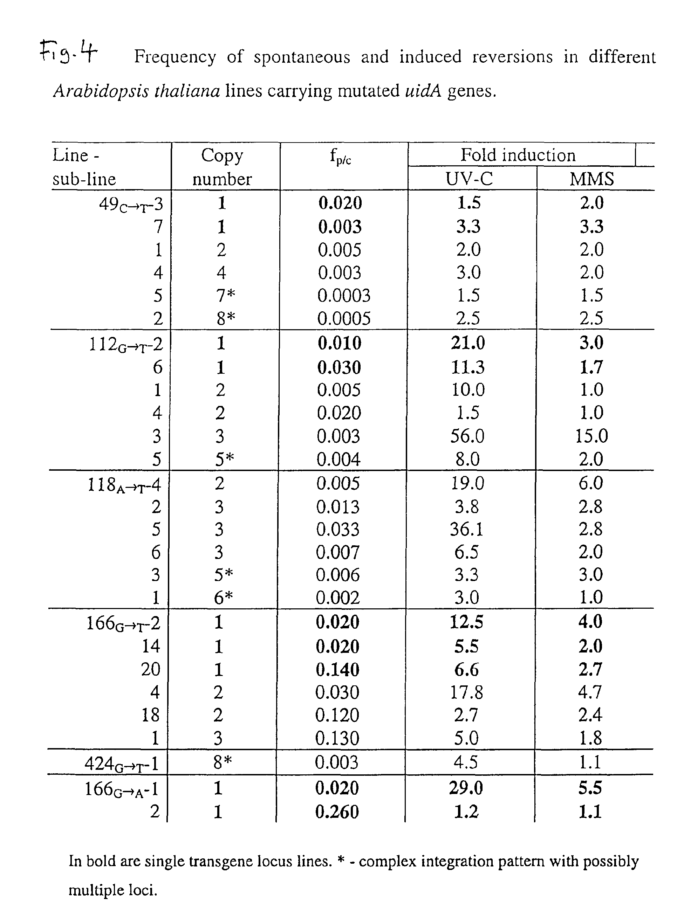

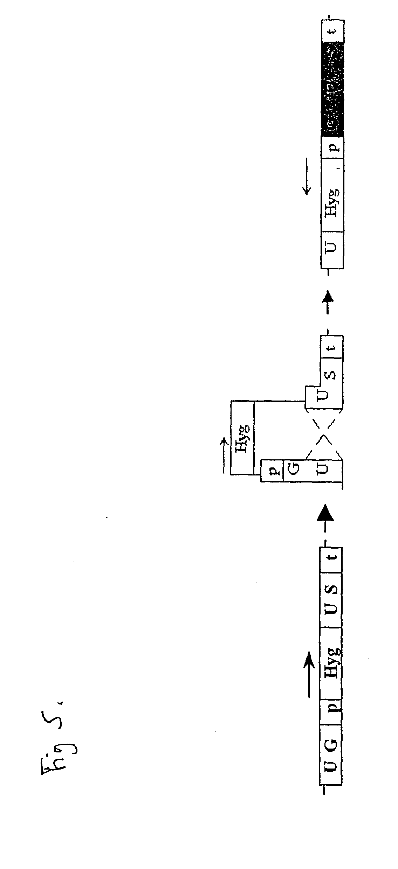

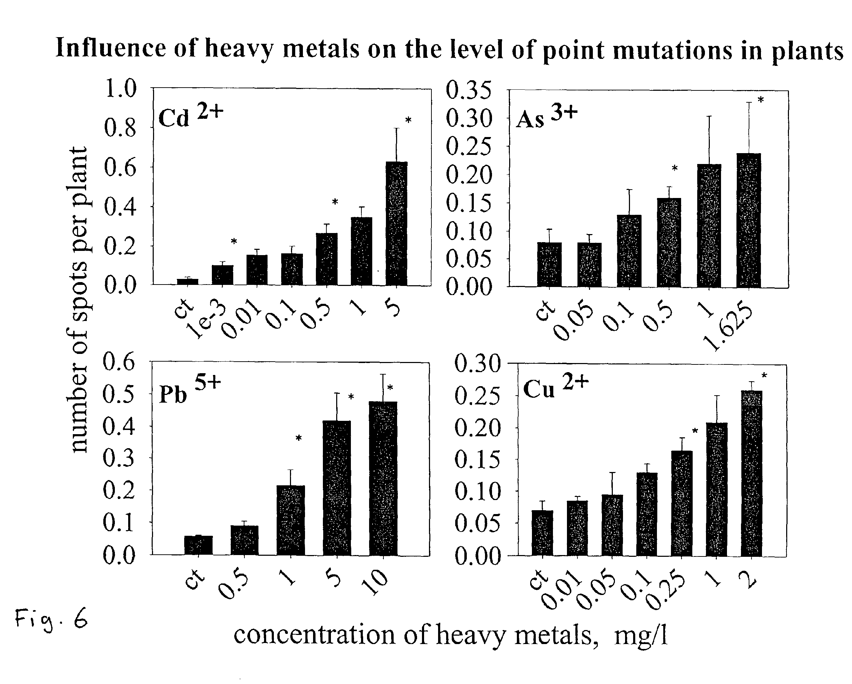

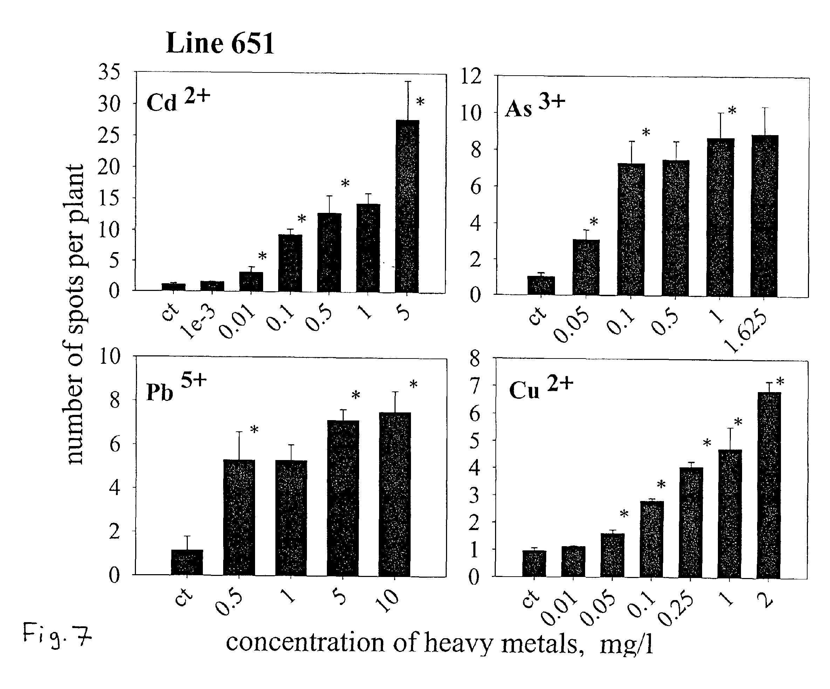

In order to evaluate whether mutations to new amino acids could restore activity of the gene, triplets with changes of single nucleotides coding for possible amino acid substitutions can be generated in place of stop codons described above (Figure 3). For this purpose, e.g. Nicotiana plumbaginifolia protoplasts can be transfected with equal amounts of DNA from different constructs. The activity of the selected gene e.g. the uidA gene can be tested using a suitable assay, in the case of uidA gene a fluorometric assay (Rossi et al., 1993). A respective experiment with the uidA gene showed that none of the amino acid substitutions gave rise to an activity higher than 2.2% of the respective non-mutated version. These background levels may be due to a low activity of the protein containing a mutated amino acid. The protein is thereby preferably activated via reversion to the original amino acid, and thus the original nucleotide will typically be found in revertants, at least at the 3 analysed positions.

For analysis of mutation frequency several homozygous lines, called sub-lines, can be generated for every mutant, e.g. uidA mutant.

### 2. Frequencies of reversions in plants such as Arabidopsis thaliana

The activity of a preferred gene can easily be monitored e.g. the uidA gene can easily be monitored by histochemical staining of plant tissue (Jefferson, 1987). Staining of whole plants can be used to reveal the presence of blue sectors on white background in uidA expressing tissue (Figure 1B). To test whether the blue sectors indeed represent reversion events, plant material from blue sectors obtained after staining can be excised from a population of plants such as those of the line 166 G→T. DNA prepared from this tissue can be used to amplify the uidA gene by Polymerase Chain Reaction (PCR). PCR products can be cloned and sequenced. In a representative experiment out of 250 independent clones prepared from plant tissue initially comprising mutated uidA gene, 98 contained reversions to the original sequence of the gene while 152 had retained the stop codon, thus reflecting the heterozygous genotype for reversion and nonsense codon, respectively. Two of the clones contained a second mutation in a codon adjacent to the reverted stop codon. Sixteen clones derived from white tissue all contained the stop-codon.

Plants from selected transgenic lines can be grown either on sterile medium or on soil under standard conditions (See Example 2). Frequencies of mutations of selected transgenic plant lines can be determined in e.g. 300-1000 histochemically stained plants per line, by relating the number of blue spots to the total number of stained plants and to the number of the transgene copies (f(p/c)-frequency per plant per one uidA gene copy) (see Example 9).

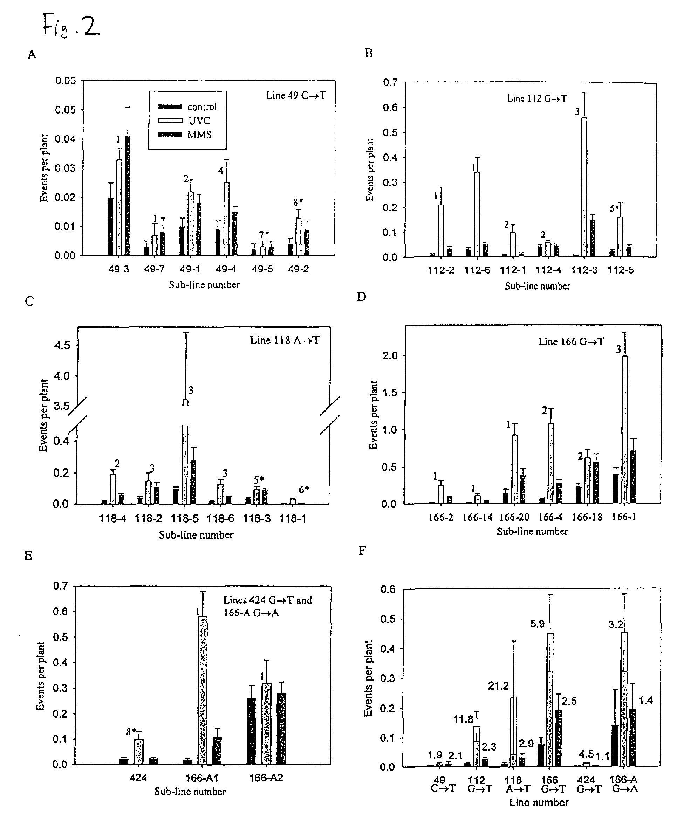

The f(p/c) between plants harboring different transgenic constructs can vary (Figure 4, Figures 2A-E). Also different sublines harboring the same transgene at presumably different chromosomal positions can exhibit a wide range of mutation frequencies. The f(p/c) was found to vary from 0.0003 to 0.14 in sub-lines 49 C→T-5 and 166 G→T-20, respectively. The frequency of reversion between sublines, in which identical single copy transgenes are located in different positions in the genome, can vary significantly. For example: two sub-lines of the 166 G→A line with one copy of the transgene each differed in reversion frequency by a factor of 13; sub-line 166 G→T-20 of line 166 G→T revealed 0.14 reversion events per plant whereas sub-line 166 G→T-2 with also one copy of the transgene reverted with f(p/c) of 0.02. Thus, reproductive material with "selected" reversion rates can be generated. Although the above figures have been obtained for uidA gene in A. thaliana, similar selected reversion rates can also be generated with other genes and/or other plants.

The higher the transgene copy number, the more possible targets exist for reversion to the dominant phenotype. Therefore, the frequency of spontaneous point mutations was expected to depend on the copy number of the analysed gene in the genome. However, it was found that a correlation between copy number and reversion frequency is not necessarily present. For example, for the uidA gene, only the 166 G→T line mutation frequencies were found to be higher in sub-lines with two and three copies of the transgene than in most of the single copy lines (Figure 4, Figure 2). For the other lines, no correlation between copy number of the mutation target and the reversion frequency could be noticed; for example, the sub-line 112 G→T-5 with 5 copies of the transgene had a frequency of reversion (0.02 spots per plant) comparable to the single-copy sub-line 112 G→T-6 (0.03 spots per plant).

Indeed, the position within a gene of the mutating bases also influences the average f(p/c). For instance, for the uidA gene, most sublines of line 49 C→T exhibited a low mutation frequency, averaging at 0.005±0.003; sub-lines 166 G→T showed the average f(p/c) of 0.076±0.024 (Figure 2F).

### 3. Reversion frequencies can be increased to different extents by e.g. UVC, MMS and/or X-ray.

To investigate whether induced DNA damage would increase the mutation frequency, several mutagens can be applied such as UV-C, X-rays and methyl methanesulfonate (MMS) to the plants carrying the nonsense mutations. Preferably mutagen doses just below those that caused visible damage are used. By the method described in example 7, it was found that irradiation of plants with UV-C (1000 J/m² for 30 s) causes a significant increase of mutation frequency in all lines (up to 56-fold), with the exception of most sub-lines of line 49 C →T, which showed a very low level of induction of mutations (Figure 2). Such differences may be explained by the specificity of the UV-C irradiation targets. The creation of CT and TT dimers at the target nucleotide may influence the mutation frequencies at that site. Indeed, reversion frequencies in lines 112 G→T, 118 A→T, 166 G→ T, in which pyrimidine dimer formation (CT or TT) between the first nucleotide in the nonsense codon (T) and the 5' adjacent nucleotide (C or T) is a possibility, are increased by exposure to UV-C to higher extents than in lines 49 C→T and 166 G→A (Figure 1A, Figure 2).

Plants grown on liquid media with MMS (50µM) were also found to exhibit increased mutation frequencies in comparison to control plants (Figure 2). Treatment with MMS e.g. stimulated the reversion frequencies by factors of 1.1 - 5.5 in different lines. In the specific experiment described below, the average level of induction of reversions varied between 1.4 and 2.9 fold and thus was most probably independent of the type of the stop-codon and the type of the mutated nucleotide (T or A). Irradiation with X-rays (e.g. absorbed dose of 25 Gy) induced mutations to a similar level namly of 1.4 to 4.8 fold.

The study of the influence of different heavy metals salts, showed that, although particular concentrations of Cu²⁺, Al³⁺, Ni²⁺ and Cd²⁺ do not cause visible phenotypic changes in exposed plants, point mutation frequency was induced. Some of the concentrations of Cu²⁺, Al³⁺, Ni²⁺ ions stated by WHO to be non toxic and non-mutagenic cause significant changes in the frequency of point mutations measured by an embodiment of the present invention. Some of the concentrations of Cu²⁺, Al³⁺, Ni²⁺ ions stated by WHO to be non-toxic and non-mutagenic cause significant changes in the frequency of point mutations (PM). For example: The highest permitted concentration of Cu²⁺ in drinking water is 1.5 mg/l; 0.25 mg/l Cu²⁺ increases PM frequency 2-3 times and 1.5 mg/l Cu²⁺ 4-6 times. The highest permitted concentration of Al³⁺ in the drinking water is 1 mg/l, this concentration increases PM frequency 2.5-3.0 times. The highest permitted concentration of Ni²⁺ in drinking water is 2 mg/l whereby 1 mg/l already increases PM frequency.

These results illustrate the advantageous, high sensitivity of the methods of the present invention.

The present invention is now further described by means of examples, that illustrate the invention but should not be construed as limiting the invention.

### Examples

### Example 1

### Generation of the stop-codon constructs

Three stop-codons were introduced by site-directed mutagenesis into 5 different positions of a 35S promoter driven β-glucuronidase gene. In order to increase the functional efficiency of the newly generated stop-codons the context of nucleotides in the neighborhood of the new stop codons was taken into consideration (Angenon et al., 1990; Stansfield et al., 1995). In all cases the base directly following the newly synthesized terminator codon was adenine or guanine. Mutations were introduced by PCR into the uidA gene contained in the pGUS23 vector (Puchta and Hohn, 1991) (Figure 1A). PCR fragments were cut with AccI endonuclease at the promoter region and with BsmI, BclI or SnaBI at the coding region and ligated into the uidA gene replacing the endogenous sequence. Mutations were confirmed by sequencing. The mutated genes were excised with EcoRI endonuclease and inserted into the binary vector pJL 513 which was partially digested with EcoRI. This vector contains as a transformation marker the bar gene, the gene coding for resistance to the herbicide "BASTA", under the control of the 1' promoter. The resulting plasmids, called pIKGUS1-6, contained six different mutations (Figure 1A).

In addition 17 mutations were generated in three different positions of the uidA gene (pIKGUS2, 3 and 4) in order to assess whether missense mutations would restore β-glucuronidase activity (Figure 3).

### Example 2

### Plant transformation and growth

Mutated uidA gene constructs cloned in E.coli-Agrobacterium shuttle vectors were mobilised into Agrobacterium tumefaciens, as described by Mattanovich et al., 1989. The resulting strains were used to transform A. thaliana ecotype Columbia, as described by Bent and Clough, 1998, with some modifications. Vacuum infiltrated plants were grown in growth chambers at 22°C with a 16 h light, 8 h dark regime until seed set. Seeds were germinated on soil and treated at the age of 7, 12 and 20 days with the herbicide BASTA (0.075%) to rescue the plants in which T-DNA integration had occurred. Pure lines homozygous for the transgenes (only single copy ones) were generated upon repeated selfing. For the estimation of spontaneous mutation frequency plants of different transgenic lines were grown on sterile MM+S media or on soil in isolated growth chambers at 22°C with 16 h light, 8 h dark regime.

### Example 3

### Histochemical staining

Histochemical staining, as described by Jefferson, 1987, was usually done with plants at the full rosette stage (4-5 weeks after germination). Plants were vacuum infiltrated 2x10 min in sterile staining buffer containing 100 mg of 5-bromo-4-chloro-3-indolyl glucuronide (X-glu, Jersey Labs Inc., USA) in 300 ml 100 mM phosphate buffer (pH 7.0), 0.05% NaN₃, 0.1% Triton X-100. Afterwards plants were incubated at 37°C for 48 hours and bleached with ethanol (FigurelB).

### Example 4

### Transient protoplast transfection

Protoplasts were prepared from leaves of Nicotiana plumbaginifolia. Equal amounts of DNA from 17 different constructs with amino acid substitutions were used for evaluation of the activity of the uidA gene. To 15 ml Falcon tubes was added in the following order: DNA 5 µg, 0.3 ml of a suspension containing 2x10⁶ protoplasts/ml, 0.3 ml 40% PEG 6000. After careful mixing the tubes were allowed to stand at room temperature for 5 min. Then 4 ml of K3 medium (Bilang et al., 1994) was added and the tubes were incubated overnight in horizontal position at 27°C in the dark. Before harvesting the transfected protoplasts, 10 ml of W5 buffer (150mM NaCl, 125mM CaCl₂, 5mM KCl, 6mM glucose) was added to each tube to dilute the high osmolarity of the K3 medium and thereby to facilitate sedimentation of the protoplasts. Protein extracts were prepared by resuspending protoplasts in 200 µl of GUS extraction buffer (50mM NaH₂PO₄ pH 7.0, 10mM EDTA, 0.1% Triton X-100, 0.1% sarcosyl). Aliquots of 20 µl were immediately taken for fluorometric reporter gene assay (Rossi et al., 1993).

### Example 5

### Southern blot analysis

Total DNA was prepared from whole Arabidopsis thaliana plants using Nucleon PhytoPure total DNA isolation kit (Amersharm Life Science) in accordance with the manufacturer's protocol. 5µg of sample DNA was digested either with HindIII (single cutter) or XhoI (non-cutter) restriction nucleases, separated on a 0.7% agarose gel and transferred onto Hybond N membranes (Amersham, Little Chalfont, UK) using manufacturer's protocol. Hybridization and washing of blots was performed according to laboratory manual (Sambrook et al., 1989). The full length GUS gene was used as a probe after ³²P-labeling with the random primer DNA labeling kit (GIBCOiBRL, Gaithersburg, MD), according to the manufacturer's instructions. For the lines used in the experiment copy numbers of the transgenes were determined.

### Example 6

### DNA amplification and sequencing

PCR analyses were performed in 50 µl volume containing 20 mM Tris (pH 8.3), 50mM KCl, 2mM MgCl2, 0.2 mM of each dNTP, 50ng template DNA, 0.25 µM of each primer, and 5U of Taq DNA polymerase. DNA amplifications were performed in a Perkin-Elmer model 9600 thermal cycler with the following profile: (i) 95°C for 5 min for 1 cycle; (ii) 94°C for 1 min, 60°C for 1 min, 72°C for 1 min for 40 cycles; and (iii) 72°C for 10 min for 1 cycle. PCR primers for site-directed mutagenesis were designed based on the uidA gene sequence.

Blue sectors were excised from about 1000 plants of sub-line 166_{G→T}-1 and were pooled. Following DNA isolation the uidA gene was amplified with the specific primers. The PCR fragments were cloned into vector pCR 2.1 (Invitrogen Corporation, CA, USA). About 250 independent clones were sequenced.

DNA sequencing reactions were performed with dRhodamine terminators from PE Applied Biosystems using a Perkin-Elmer GeneAmp PCR system 9600 thermocycler and analysed using an ABI PRISM 377 DNA sequencer.

### Example 7

### Treatment of Arabidopsis seedlings with UV, X-rays, and MMS

Two week old Arabidopsis seedlings grown on soil were irradiated with 1000 J/m², 30 s of UV-C light (254 nm) using a Minerallight-Lamp R-52 (UV-Products Inc., San Gabriel, CA, USA) or with 25 Gy X-rays.

Plants of the same age but grown on sterile medium were transferred into liquid medium containing or lacking 50 µM MMS. Two weeks later the plants were histochemically stained and blue spots were counted.

### Example 8

### Calculation of the number of genomes per plant

Total DNA of the respective transgenic lines was isolated from whole plants at the full rosette stage, using the Nucleon phytopure plant DNA extraction kit from Amersham Life Science. The yield of total DNA (in micrograms per plant) was compared with the mean DNA content (0.28 picograms) of a diploid A. thaliana cell, to give an estimate of 2x10⁶ - 8x10⁶ (dependent on DNA preparation) genomes per plant (Swoboda et al., 1994).

### Example 9

### Calculation of the reversion frequency

To evaluate mutation frequency per plant per copy number the following formula was used:

f(p/c) = r(p)/n(c), whereby f(p/c) is the mutation frequency per plant per copy of the transgene, r(p) is the number of reversion events per plant, as an average of 300-1000 plants, n(c) is the number of transgene copies in the genome.

For evaluation of the mutation frequency per bp the following formula was used:

f(b)= r(p)/n(c)x 2n(g), whereby f(b) is the mutation frequency per bp, r(p), n(c) as described above, n(g) is the number of haploid genomes present in Arabidopsis thaliana plants at the stage of analysis.

### Example 10

### Growth of point mutation plants on metal containing media and measurement of induced mutations

For the estimation of metal induced mutation frequencies sterilised seeds of homozygous transgenic A. thaliana plants of lines 166 G-T and 166 G-A were planted on media containing various concentrations of different heavy metals (Cd, Pb, Cu) and metalloid elements (As) in isolated growth chambers at 22°C with 16 h light, 8 h dark regime for 35 days. Point mutation frequencies were obtained by counting mutation events (sectors) in each plant, separately summing and relating these data to the number of plants in the population. For each experimental condition 1000 plants were scored. The experiments were repeated 3 times and the mean values of point muatation frequencies for plant populations for each condition were calculated (Figure 6).

### Example 11

### Recombination assay for determination of heavy metal induced mutations

The homozygous transgenic Arabidopsis thaliana plant-line 651 was used to study heavy metal induced recombination frequency (Puchta H et al., 1995). These plants carried one copy per haploid genome of overlapping, non-functional, truncated version of a chimeric β-glucuronidase (uidA) marker gene as a recombination substrate, with 566 bp overlapping sequences in inverted orientation (Figure 5). Upon homologous recombination the gene was restored and upon histochemical staining the recombination events were scored as blue sectors on the white plants, enabling conduction of a quantitative assay.

Seeds of line 651 were planted on media containing various concentrations of different heavy metals (Cd, Pb, Cu) and metalloid elements (As) in isolated growth chambers at 22°C with 16 h light, 8 h dark regime for 35 days.

Recombination frequencies were obtained by counting recombination events (sectors) in each plant, seperately summing and relating these data to the number of plants in the population. RF=X/N, were RF is recombination frequency, X is number of recombination events in a plant population and N is the number of plants in a population. For each experimental condition 200 plants were scored. The experiments were repeated 6 times and the mean values of recombination frequencies for plant populations for each condition were calculated (Figure 7).

While there are shown and described presently preferred embodiments of the invention, it is to be distinctly understood that the invention is not limited thereto but may be otherwise variously embodied and practised within the scope of the following claims. Any publications referred to herein are incorporated by reference in their entirety as if referred to individually.

### References.

Angenon, G. *et al.* (1990) Analysis of the stop codon context in plant nuclear genes. *FEBS Lett.* 271, 144-146.

Atkinson, J. and Martin, R. (1994) Mutations to nonsense codons in human genetic disease: implications for gene therapy by nonsense suppressor tRNAs. *Nucleic Acids Res.,* 22, 1327-1334.

Bent, A., Clough, S. (1998) *Agrobacterium* germ-line transformation: transformation of *Arabidopsis* without tissue culture. *Plant Mol. Biol. Manual,* B7, 1-14.

Betzner, A. *et al*. (1997) Transfer RNA-mediated suppression of amber stop codons in transgenic *Arabidopsis thaliana*. *Plant J.,* 11, 587-595.

Bilang, R. *et al.* (1994) PEG-mediated direct gene transfer and electroporation. *Plant Mol. Biol. Manual,* A1, 1-16.

Jefferson, R. (1987) Assaying chimeric genes in plants: The GUS gene fusion system. *Plant Mol. Biol. Reporter,* 5, 387-405.

Kovalchuk, I., Kovalchuk O., Arkhipov A., Hohn B. (1998) Transgenic plants are sensitive bioindicators of nuclear pollution caused by the Chernobyl accident. Nature Biotechnology 16, 1054-1059.

Kovalchuk, I., Kovalchuk, O., Hohn, B. (1999) Transgenic plants as bioindicators of environmental pollution. AgBiotechNet, 1, 1-6.

Mattanovich, D. *et al.* (1989) Efficient transformation of Agrobacterium spp. by electroporation. *Nucleic Acids Res.,* 17, 6747.

Puchta, H., Hohn, B. (1991) A transient assay in plant cells reveals a positive correlation between extrachromosomal recombination rates and length of homologous overlap. *Nucl. Acids Res.,* 19, 2693-2700.

Puchta, H., Swoboda, P., and Hohn, B. (1995) Induction of homologous DNA recombination in whole plants. Plant J., 7, 203-210.

Rossi, L. et al. (1993) Efficient and sensitive assay for T-DNA-dependent transient gene expression. *Plant Mol. Biol. Reporter,* 11, 220-229.

Sambrook, J. *et al.* (1989) Molecular cloning: A laboratory manual. 2nd edn. Cold Spring Harbor Laboratory Press, Cold Spring Harbor, NY.

Stadler, L. (1930) The frequency of mutation of specific genes in maize (Abstr.). *Anat.Rec*. *47,* 381.

Stansfield, I. *et al.* (1995) The end in sight: terminating translation in eukaryotes. *TIBS*, 20, 489-491.

Swoboda, P. *et al*. (1994) Intrachromosomal homologous recombination in whole plants. EMBO J. 13, 484-489.

## Claims

1. Process for monitoring mutagens, **characterized in that** said process comprises
a) contacting a plant cell or protoplast with a mutagen, said plant cell or protoplast comprising a nucleic acid with at least one point mutation resulting in a detectable change in function and/or expression of a reporter molecule and
b) detecting the presence of a functional reporter molecule as an indicator or reversion of said mutation.

2. The process of claim 1, wherein said nucleic acid due to said at least one point mutation, encodes a non-functional reporter molecule.

3. The process of claim 1, wherein said plant or protoplast comprises as nucleic acid with at least one point mutation a nucleic acid encoding a repressor that due to said point mutation has reduced or no repressor activity, whereby said repressor expressed in the absence of said point mutation is a repressor inhibiting expression of said reporter molecule.

4. The process of claim 2, wherein said nucleic acid encodes a reporter molecule that comprises one intron in the reporter-coding sequence, wherein said at least one point mutation introduces in said intron or an intron-exon boundary an alternative splice site and thereby affects expression of said reporter molecule.

5. The process of claim 1 that comprises as nucleic acid with at least one point mutation a promoter that is operably linked to said reporter molecule, whereby said mutation in said promoter affects expression of said reporter molecule.

6. Process according to anyone of claims 1 to 5 **characterized in that** said plant cell or protoplast stems from a plant selectecd from the group consisting of Arabidopsis species, Brassica species, tobacco, wheat, maize, rice, barley, Nicotiana.

7. Process according to anyone of the preceding claims, **characterized in that** said nucleotide sequence encoding said reporter gene is selected from the group consisting of β-glucuronidase, galactosidase, Green Fluorescent Protein, luciferase, R locus in maize, herbicide resistance, fungicide resistance and antibiotic resistance.

8. Process according to anyone of the preceding claims **characterized in that** the mutation in the nucleotide sequence encoding a functional reporter protein leads to an amino acid substitution, in particular to a stop codon.

9. Process according to claim 8 **characterized in that** the mutation is selected from the group comprising the nucleotide changes C to A, G to T, A to T, G to A and mixtures thereof.

10. Process according to anyone of the preceding claims, **characterized in that** the reversion of the nucleotide sequence encoding a non functional reporter protein to a sequence encoding a functional reporter protein is detected by spectrophotometric, fluorometric, histochemical methods or selection for resistance or survival.

11. Process according claim 1 or 3 **characterized in that** said repressor system has eukaryotic or prokaryotic origin.

12. Process according to claim 11 **characterized in that** said prokaryotic repressor system is the Tet repressor system or the LacI/LacZ system.

13. Process according to claim 1 or 4 **characterized in that** the mutations introduced in said intron and/or intron-exon boundary of said intron abolish splicing.

14. Process according to claim 1 or 5 **characterized in that** said promoter operably linked to said reporter molecule is selected from the group consisting of viral e.g. CaMV 35S, 19S, Ti plasmid e.g. nopaline synthase, plant e.g. actin, ubiquitin.

15. A plant, protoplast or a reproduction material having a nucleic acid with at least one point mutation resulting in a detectable change in function and/or expression of a reporter meolecule.

16. The plant, protoplast or reproduction material of claim 15, wherein said nucleic acid due to said at least one point mutation, encodes a non-functional reporter molecule.

17. The plant, protoplast or reproduction material of claim 15 that comprises as nucleic acid with at least one point mutation a nucleic acid encoding a repressor that due to said point mutation has reduced or no repressor activity, whereby said repressor expressed in the absence of a point mutation is a repressor inhibiting expression of said reporter molecule.

18. The plant, protoplast or reproduction material of claim 16, wherein said nucleic acid encodes a reporter molecule that comprises one intron in the reporter-coding sequence, wherein said at least one point mutation introduces in the intron or intron-exon boundary an alternative splice site and thereby affects expression of said reporter molecule.

19. The plant, protoplast or reproduction material of claim 15 that comprises as nucleic acid with at least one point mutation a promoter that is operably linked to said reporter molecule, whereby said mutation in said promoter sequence affects expression of said reporter molecule.

20. The plant or reproduction material according to anyone of claims 15 to 19 **characterized in that** said plant is selected from the group consisting of Arabidopsis sp., Brassica sp., wheat, maize, rice, Nicotiana, barley.

21. The plant or reproduction material according to claim 15 to 20, **characterized in that** said nucleotide sequence encoding said reporter protein is selected from the group consisting of β-glucuronidase, galactosidase, Green Fluorescent Protein, luciferase, R locus in maize, herbicide resistance, fungicide resistance, and antibiotic resistance.

22. The plant or reproduction material according to claim 21, **characterized in that** said mutation in the nucleotide sequence encoding a functional reporter protein leads to a amino acid substitution, in particular to a stop codon.

23. The plant or reproduction material according to claim 22, **characterized in that** said mutation is selected from the group comprising the nucleotide changes C to A, G to T, A to T, G to A and mixtures thereof.

24. The plant or reproduction material according to claim 15 or 17, **characterized in that** said repressor system has eukaryotic or prokaryotic origin.

25. The plant or reproduction material according to claim 24 **characterized in that** said prokaryotic repressor system is the Tet repressor system or the LacI/LacZ system.

26. The plant or reproduction material according to claim 15 or 18 **characterized in that** the mutations introduced in the intron-exon boundary of said intron abolish splicing.

27. The plant or reproduction material according to claim 15 or 19, **characterized in that** said promoter operably linked to said reporter molecule is selected from the group consisting of viral e.g. CaMV 35S, 19S, Ti plasmid e.g. nopaline synthase, plant e.g. actin, ubiquitin.

28. Use of a plant according to anyone of claims 15 to 27 for toxicity tests or as bioindicator of environmental pollution.

29. Use of a plant for monitoring mutagens others than radiation, said plant comprising a non-functional marker gene serving as a recombination substrate that upon mutagen induced recombination regains functionality.

30. Use of a plant for monitoring mutagens, said plant comprising a non-functional marker gene serving as a recombination substrate that upon mutagen induced recombination regains functionality in combination with a plant according to anyone of claims 15 to 26.
